# EUROPÄISCHE PATENTSCHRIFT

(11) **EP 3 272 728 B1**
(45) Veröffentlichungstag und Bekanntmachung des Hinweises auf die Patenterteilung: **19.06.2019**
(21) Anmeldenummer: 16180052.9
(22) Anmeldetag: 19.07.2016
(51) Int. Cl.: C07C 67/36, C07C 69/24, C07C 69/612

(54) **VERFAHREN ZUR ALKOXYCARBONYLIERUNG VON ALKOHOLEN**
METHOD FOR THE ALCOXYCARBONYLATION OF ALCOHOLS
PROCEDE D'ALKOXY-CARBONYLATION D'ALCOOLS

(43) Veröffentlichungstag der Anmeldung: 24.01.2018
(73) Patentinhaber: Evonik Degussa GmbH, 45128 Essen (DE)
(72) Erfinder: DONG, Kaiwu, 236800 Bo Zhou (CN); JACKSTELL, Ralf, 27478 Cuxhaven Altenwalde (DE); NEUMANN, Helfried, 18055 Rostock (DE); BELLER, Matthias, 18211 Ostseebad Nienhagen (DE); FRIDAG, Dirk, 45721 Haltern am See (DE); HESS, Dieter, 45770 Marl (DE); DYBALLA, Katrin Marie, 45657 Recklinghausen (DE); GEILEN, Frank, 45721 Haltern am See (DE); FRANKE, Robert, 45772 Marl (DE)

(56) Entgegenhaltungen:
- WO-A1-2011/083305
- ZHOU H ET AL: "Catalytic carbonylation of alpha-(6-methoxyl-2-naphthyl)ethanol to methyl esters of naproxen using PdCl2-CuCl2-PPh3-acid catalyst system", JOURNAL OF ORGANOMETALLIC CHEMISTRY, ELSEVIER-SEQUOIA S.A. LAUSANNE, CH, Bd. 556, Nr. 1-2, 15. April 1998 (1998-04-15), Seiten 239-242, XP004127950, ISSN: 0022-328X, DOI: 10.1016/S0022-328X(97)00757-2
- W. CLEGG; G. R. EASTHAM; M. R. J. ELSEGOOD; R. P. TOOZE; X. L. WANG; K. WHISTON: CHEM. COMMUN, 1999, Seiten 1877-1878, XP002466911,

## Beschreibung

Die vorliegende Erfindung betrifft ein neues Verfahren zur Alkoxycarbonylierung von Alkoholen.

Die Alkoxycarbonylierung ethylenisch ungesättigter Verbindungen ist ein bekanntes Verfahren zur Herstellung von Estern. Bei diesem Verfahren werden ethylenisch ungesättigte Verbindungen (Olefine) mit Kohlenmonoxid und Alkoholen in Gegenwart eines Metall-Ligand-Komplexes zu den entsprechenden Estern umgesetzt. Üblicherweise wird als Metall Palladium eingesetzt. Das folgende Schema zeigt die allgemeine Reaktionsgleichung einer Alkoxycarbonylierung:

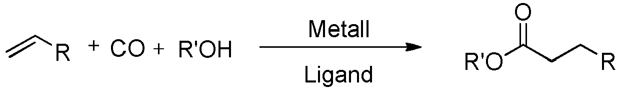

Ein sehr gutes katalytisches System für dieses Verfahren wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

In ZHOU H ET AL, "Catalytic carbonylation of alpha-(6-methoxyl-2-naphthyl)ethanol to methyl esters of naproxen using PdCl2-CuCl2-PPh3-acid catalyst system", JOURNAL OF ORGANOMETALLIC CHEMISTRY, ELSEVIER-SEQUOIA S.A. LAUSANNE, CH, (1998-04-15), Bd. 556, Nr. 1-2, Seiten 239 - 242, wird ein Verfahren beschrieben, mit welchem alpha-(6-Methoxyl-2-naphthyl)ethanol zu dem entsprechenden Methylester umgewandelt wird.

Es ist bislang nicht bekannt, dass andere Verbindungen als ethylenisch ungesättigte Verbindungen mit Alkoholen und CO zu den entsprechenden Estern in Form einer Alkoxycarbonylierungsreaktions umgesetzt werden können.

Vor diesem Hintergrund ist es Aufgabe der vorliegenden Erfindung, ein Alkoxycarbonylierungsverfahren zur Herstellung von Estern bereit zu stellen, bei dem als Ausgangsprodukt an Stelle von ethylenisch ungesättigten Verbindungen andere Rohstoffe verwendet werden. Von besonderem Interesse ist dabei der Einsatz von Alkoholen als Ausgangsprodukt für die Alkoxycarbonylierung.

Es hat sich überraschenderweise gezeigt, dass diese Aufgabe durch ein Alkoxycarbonylierungsverfahren gelöst wird, bei dem besondere Benzol-basierte Diphosphinliganden, bei denen mindestens eine Phosphingruppe mit einem Heteroarylrest substituiert ist, eingesetzt werden. Das erfindungsgemäße Verfahren zeichnet sich durch hohe Ausbeuten bezogen auf die als Edukt eingesetzten Alkohole aus.

Die Erfindung betrifft somit ein Verfahren umfassend die Verfahrensschritte:
a) Vorlegen eines ersten Alkohols, wobei der erste Alkohol 2 bis 30 Kohlenstoffatome aufweist;
b) Zugabe eines Phosphinliganden und einer Verbindung, welche Pd umfasst,
   oder Zugabe eines Komplexes umfassend Pd und einen Phosphinliganden;
c) Zugabe eines zweiten Alkohols;
d) Zuführen von CO;
e) Erwärmen des Reaktionsgemisches, wobei der erste Alkohol mit CO und dem zweiten Alkohol zu einem Ester umgesetzt wird;
wobei der Phosphinligand eine Verbindung gemäß Formel (**I**) ist 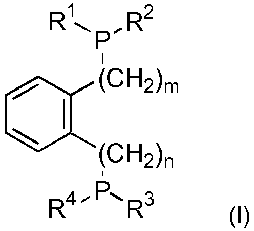 wobei
m und n unabhängig voneinander jeweils 0 oder 1 sind;
R¹, R², R³, R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
mindestens einer der Reste R¹, R², R³, R⁴ für einen -(C₃-C₂₀)-Heteroarylrest steht;
   und
R¹, R², R³, R⁴, falls diese für -(C₁-C₁₂)-Alkyl, -(C₃-C₁₂)-Cycloalkyl, -(C₃-C₁₂)-Heterocycloalkyl,-(C₆-C₂₀)-Aryl, oder -(C₃-C₂₀)-Heteroaryl stehen,
jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl,-CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können, und
wobei der erste Alkohol in Verfahrensschritt a) ein sekundärer oder tertiärer Alkohol ist und der zweite Alkohol in Verfahrensschritt c) ein primärer Alkohol ist,
hierbei umfasst der Begriff (C₃-C₁₂)-Heterocycloalkyl nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind.

Bei dem ersten Alkohol handelt es sich um einen sekundären oder tertiären Alkohol und bei dem zweiten Alkohol um einen primären Alkohol.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

In einer Ausführungsform handelt es sich bei den erfindungsgemäßen Phosphinliganden um Verbindungen gemäß einer der Formeln (**II**) und (**III**)

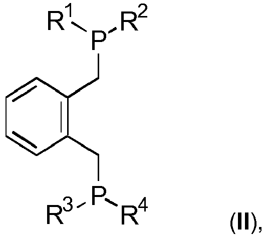

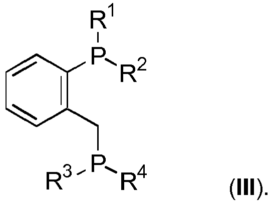

Dabei haben die Reste R¹, R², R³, R⁴ jeweils die oben genannte Bedeutung.

In einer besonders bevorzugten Ausführungsform handelt es sich bei dem erfindungsgemäßen Phosphinliganden um eine Verbindung der Formel (**II**), wobei die Reste R¹, R², R³, R⁴ die oben genannte Bedeutung haben.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-CyCloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.

In einer Ausführungsform können die Reste R¹, R², R³, R⁴, falls sie für -(C₁-C₁₂)-Alkyl, , -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H,-NH₂, Halogen substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃₋C₂₀)-Heteroaryl-(C₁-C₁₂)-Alkyl, -(C₃-C₂₀)-Heteroaryl-O-(C₁-C₁₂)-Alkyl, substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl oder -(C₃-C₂₀)-Heteroaryl stehen, jeweils unabhängig voneinander mit einem oder mehreren Substituenten ausgewählt aus -(C₁-C₁₂)-Alkyl, -O-(C₁-C₁₂)-Alkyl-(C₆-C₂₀)-Aryl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl und -(C₃-C₂₀)-Heteroaryl substituiert sein.

In einer Ausführungsform sind die Reste R¹, R², R³, R⁴ unsubstituiert, falls sie für -(C₁-C₁₂)-Alkyl, -(C₃-C₁₂)-Cycloalkyl, oder -(C₃-C₁₂)-Heterocycloalkyl stehen, und können wie beschrieben substituiert sein, falls sie für -(C₆-C₂₀)-Aryl, oder -(C₃-C₂₀)-Heteroaryl stehen.

In einer Ausführungsform sind die Reste R¹, R², R³, R⁴ unsubstituiert, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen.

In einer Ausführungsform sind R¹, R², R³, R⁴ jeweils unabhängig voneinander ausgewählt aus -(C₁-C₁₂)-Alkyl, -(C₆-C₂₀)-Aryl, -(C₃-C₂₀)-Heteroaryl;
wobei mindestens einer der Reste R¹, R², R³, R⁴ für einen -(C₃-C₂₀)-Heteroarylrest steht; und R¹, R², R³, R⁴, falls diese für -(C₁-C₁₂)-Alkyl, -(C₆-C₂₀)-Aryl oder -(C₃-C₂₀)-Heteroaryl stehen, jeweils unabhängig voneinander mit einem oder mehreren der oben beschriebenen Substituenten substituiert sein können.

In einer Ausführungsform stehen mindestens zwei der Reste R¹, R², R³, R⁴ für einen -(C₃-C₂₀)-Heteroaryl rest.

In einer Ausführungsform stehen die Reste R¹ und R³ jeweils für einen -(C₃-C₂₀)-Heteroarylrest und können jeweils unabhängig voneinander mit einem oder mehreren der oben beschriebenen Substituenten substituiert sein. Vorzugsweise sind R² und R⁴ dabei unabhängig voneinander ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl, besonders bevorzugt aus -(C₁-C₁₂)-Alkyl, -(C₃-C₁₂)-Cycloalkyl, -(C₆-C₂₀)-Aryl, am meisten bevorzugt aus -(C₁-C₁₂)-Alkyl. R² und R⁴ können dabei unabhängig voneinander mit einem oder mehreren der oben beschriebenen Substituenten substituiert sein.

In einer Ausführungsform stehen die Reste R¹, R², R³ und R⁴ für einen -(C₆-C₂₀)-Heteroarylrest und können jeweils unabhängig voneinander mit einem oder mehreren der oben beschriebenen Substituenten substituiert sein.

In einer Ausführungsform sind die Reste R¹, R², R³ und R⁴, falls sie für einen Heteroarylrest stehen, jeweils unabhängig voneinander ausgewählt aus Heteroarylresten mit fünf bis zehn Ringatomen, vorzugsweise fünf oder sechs Ringatomen.

In einer Ausführungsform stellen die Reste R¹, R², R³ und R⁴, falls sie für einen Heteroarylrest stehen, einen Heteroarylrest mit fünf Ringatomen dar.

In einer Ausführungsform sind die Reste R¹, R², R³ und R⁴, falls sie für einen Heteroarylrest stehen, jeweils unabhängig voneinander ausgewählt aus Heteroarylresten mit sechs bis zehn Ringatomen.

In einer Ausführungsform stellen die Reste R¹, R², R³ und R⁴, falls sie für einen Heteroarylrest stehen, einen Heteroarylrest mit sechs Ringatomen dar.

In einer Ausführungsform sind die Reste R¹, R², R³ und R⁴, falls sie für einen Heteroarylrest stehen, ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl, wobei die genannten Heteroarylreste wie oben beschrieben substituiert sein können.

In einer Ausführungsform sind die Reste R¹, R², R³ und R⁴, falls sie für einen Heteroarylrest stehen, ausgewählt aus Furyl, Thienyl, Pyrrolyl, Imidazolyl, Pyridyl, Pyrimidyl, Indolyl, wobei die genannten Heteroarylreste wie oben beschrieben substituiert sein können.

In einer Ausführungsform sind die Reste R¹, R², R³ und R⁴, falls sie für einen Heteroarylrest stehen, ausgewählt aus 2-Furyl, 2-Thienyl, 2-Pyrrolyl, 2-lmidazolyl, 2-Pyridyl, 2-Pyrimidyl, 2-Indolyl, wobei die genannten Heteroarylreste wie oben beschrieben substituiert sein können.

In einer Ausführungsform sind die Reste R¹, R², R³ und R⁴, falls sie für einen Heteroarylrest stehen, ausgewählt aus 2-Furyl, 2-Thienyl, N-Methyl-2-Pyrrolyl, N-Phenyl-2-Pyrrolyl, N-(2-Methoxyphenyl)-2-Pyrrolyl, 2-Pyrrolyl, N-Methyl-2-Imidazolyl, 2-Imidazolyl, 2-Pyridyl, 2-Pyrimidyl, N-Phenyl-2-Indolyl, 2-Indolyl, wobei die genannten Heteroarylreste nicht weiter substituiert sind.

Besonders bevorzugt stellen die Reste R¹, R², R³ und R⁴, falls sie für einen Heteroarylrest stehen, Pyridyl dar, insbesondere 2-Pyridyl.

In einer Ausführungsform stellen R¹ und R³ einen Pyridylrest, bevorzugt 2-Pyridyl, dar und R² und R⁴ stehen für -(C₁-C₁₂)-Alkyl, wobei R¹, R², R³ und R⁴ jeweils wie oben beschrieben substituiert sein können.

In einer Ausführungsform handelt es sich bei dem Phosphinliganden um eine Verbindung gemäß Formel (1):

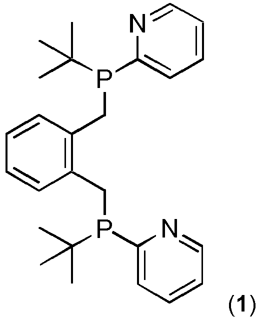

Die in dem erfindungsgemäßen Verfahren in Schritt a) als Edukt eingesetzten Alkohole umfassen 2 bis 30 Kohlenstoffatome, bevorzugt 3 bis 22 Kohlenstoffatome, besonders bevorzugt 4 bis 12 Kohlenstoffatome. Es kann sich dabei um primäre, sekundäre oder tertiäre Alkohole handeln. Die Alkohole können auch aromatische Gruppen umfassen.

Vorzugsweise handelt es sich bei dem in Schritt a) eingesetzten Alkohol um einen sekundären oder tertiären Alkohol.

In einer Ausführungsform ist der in Schritt a) eingesetzte erste Alkohol eine Verbindung gemäß der Formel (**IV**) 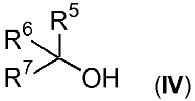
wobei R⁵ ausgewählt ist aus -(C₁-C₁₂)-Alkyl, -(C₃-C₁₂)-Cycloalkyl, -(C₆-C₂₀)-Aryl;
und R⁶ und R⁷ jeweils unabhängig voneinander ausgewählt sind aus -H, -(C₁-C₁₂)-Alkyl, -(C₃-C₁₂)-Cycloalkyl, -(C₆-C₂₀)-Aryl,
wobei höchstens einer der Reste R⁶ und R⁷ -H ist.

In einer bevorzugten Ausführungsform handelt es sich bei R⁵ um -(C₁-C₁₂)-Alkyl. Vorzugsweise ist R⁵ ausgewählt aus Methyl, Ethyl, n-Propyl, 2-Propyl, n-Butyl, 2-Butyl, *sec*-Butyl, *tert*-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl. Besonders bevorzugt ist R⁵ ausgewählt aus Methyl und Ethyl. Am meisten bevorzugt handelt es sich bei R⁵ um Methyl.

In einer bevorzugten Ausführungsform sind R⁶ und R⁷ jeweils unabhängig voneinander ausgewählt aus -H, -(C₁-C₁₂)-Alkyl und -(C₆-C₂₀)-Aryl, wobei höchstens einer der Reste R⁶ und R⁷ -H ist.

Vorzugsweise sind R⁶ und R⁷ jeweils unabhängig voneinander ausgewählt aus -H, Methyl, Ethyl, n-Propyl, 2-Propyl, n-Butyl, 2-Butyl, *sec*-Butyl, *tert*-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl und Phenyl, wobei höchstens einer der Reste R⁶ und R⁷ -H ist.

Besonders bevorzugt sind R⁶ und R⁷ jeweils unabhängig voneinander ausgewählt aus -H, Methyl, Ethyl, n-Propyl, 2-Propyl, n-Butyl, 2-Butyl, sec-Butyl, *tert*-Butyl und Phenyl, wobei höchstens einer der Reste R⁶ und R⁷ -H ist.

In einer Ausführungsform ist R⁵ Methyl und R⁶, R⁷ sind unabhängig voneinander ausgewählt aus -H, Methyl, *tert*-Butyl und Phenyl, wobei höchstens einer der Reste R⁶, R⁷ -H darstellt.

In einer alternativen Ausführungsform sind R⁶ und R⁷ jeweils unabhängig voneinander ausgewählt aus -(C₁-C₁₂)-Alkyl und -(C₆-C₂₀)-Aryl. Vorzugsweise sind R⁶ und R⁷ hierbei jeweils unabhängig voneinander ausgewählt aus Methyl, Ethyl, n-Propyl, 2-Propyl, n-Butyl, 2-Butyl, *sec*-Butyl, *tert*-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl und Phenyl.

Besonders bevorzugt sind R⁶ und R⁷ hierbei jeweils unabhängig voneinander ausgewählt aus Methyl, Ethyl, n-Propyl, 2-Propyl, n-Butyl, 2-Butyl, *sec*-Butyl, *tert*-Butyl und Phenyl. Insbesondere kann in dieser Ausführungsform R⁵ Methyl sein, wobei R⁶ und R⁷ jeweils unabhängig voneinander ausgewählt sind aus Methyl, *tert*-Butyl und Phenyl.

In einer bevorzugten Ausführungsform ist der erste Alkohol ausgewählt aus *tert*-Butanol, 3,3-Dimethylbutan-2-ol und 1-Phenyl-ethanol.

Die erfindungsgemäße Alkoxycarbonylierung wird durch einen Pd-Komplex katalysiert. Der Pd-Komplex kann dabei in Verfahrensschritt b) entweder als präformierter Komplex umfassend Pd und den Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

Die präformierten Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Acetat-Anionen, Maleinimide (z.B. N-Methylmaleinimid), 1,4-Naphthochinon, Trifluoroacetat-Anionen oder Chloridanionen.

In dem Fall, dass der Katalysator *in situ* gebildet wird, kann der Ligand im Überschuss zugegeben werden, so dass im Reaktionsgemisch auch ungebundener Ligand vorliegt.

Auch im Falle des Komplexes, der gleich zu Beginn zugesetzt wird, kann zusätzlich weiterer Ligand zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂]

Vorzugsweise handelt es sich bei der Verbindung, welche Pd umfasst, um PdCl₂, Pd(acac)₂ oder Pd(OAc)₂. Besonders geeignet ist Pd(acac)₂.

Der zweite Alkohol in Verfahrensschritt c) kann verzweigt oder linear sein, cyclisch, alicyclisch oder teilweise zyklisch und stellt insbesondere ein C₁- bis C₃₀-Alkanol dar. Es können Monoalkohole oder Polyalkohole verwendet werden.

Vorzugsweise wird ein aliphatischer Alkohol eingesetzt. Als aliphatischer Alkohol wird im Rahmen dieser Erfindung ein Alkohol bezeichnet, der keine aromatischen Gruppen umfasst, also beispielsweise ein Alkanol, Alkenol oder Alkinol. Somit sind auch ungesättigte, nichtaromatische Alkohole zugelassen.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl-, Amino-, Ether-, Thioether-, oder Silylgruppen, und/oder Halogensubstituenten enthalten.

In einer Ausführungsform umfasst der Alkohol keine weiteren funktionellen Gruppen außer Hydroxylgruppen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, 1-Butanol, 1-Pentanol, 1-Hexanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol und 1,1,1-Tri(hydroxymethyl)ethan.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus Methanol und Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um den Alkohol zu einem Ester umzusetzen.

Das molare Verhältnis des in Schritt a) vorgelegten Alkohols zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu dem in Schritt a) vorgelegten Alkohol beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis des Phosphinliganden zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 4 mol-%, bezogen auf die Stoffmenge des in Schritt a) eingesetzten Alkohols.

Dem Reaktionsgemisch kann außerdem ein Molekularsieb zugesetzt werden. diese Maßnahme erhöht in einigen Fällen die Ausbeute an Ester. Zu diesem Zweck eignen sich beispielsweise Molekularsiebe aus Zeolith oder aus Kohlenstoff, vorzugsweise Molekularsiebe aus Zeolith. Vorzugsweise liegt die Porenweite des Molekularsiebs im Bereich von 1 bis 10 Å, besonders bevorzugt im Bereich von 2 bis 6 Å, am meisten bevorzugt im Bereich von 3 bis 5 Å.

### Beispiele

Die folgenden Beispiele veranschaulichen die Erfindung.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} ^{∗} (BF_{31P} / BF_{1H}) = SR_{1H} ^{∗} 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Darstellung von Chlor-2-pyridyl-tert-butylphosphin (Vorstufe A)

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

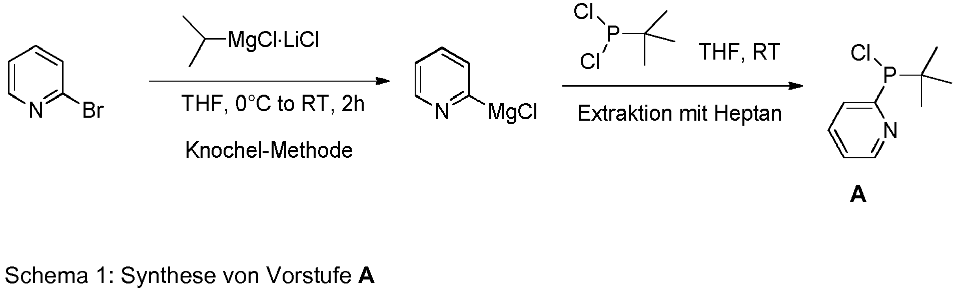

In einen 50 ml-Rundkolben mit Magnetrührer und Septum werden unter Argon 8,07 ml einer 1,3 M Isopropylmagnesium-chloridlösung (Knochel-Reagenz) gegeben und auf -15 °C gekühlt. Danach werden zügig 953.5 µl (10 mmol) 2-Bromopyridin zugetropft. Die Lösung wird sofort gelb. Man lässt auf -10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1,748 g (11 mmol) Dichloro-tert-butylphosphin in 10 ml THF, die vorher auf -15 °C gekühlt worden ist, mit einer Spritzenpumpe langsam zugetropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht Rühren. Laut GCMS hat sich viel Produkt gebildet. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4,6 mbar, 120 °C Ölbad und 98 °C Übergangstemperatur kann das Produkt destilliert werden. Man erhält 1,08 g eines farblosen Öls. (50%).
Analytische Daten: ¹H NMR (300 MHz, C₆D₆): δ 8,36 (m, 1H, Py), 7,67 (m, 1H, Py), 7,03-6,93 (m, 1H, Py), 6,55-6,46 (m, 1H, Py), 1,07 (d, J = 13,3 Hz, 9H, t-Bu).
¹³C NMR (75 MHz, C₆D₆): δ 162,9, 162,6, 148,8, 135,5, 125,8, 125,7, 122,8, 35,3, 34,8, 25,9 und 25,8.
³¹P NMR (121 MHz, C₆D₆) δ 97,9.
MS (EI) *m*:*z* (relative Intensität) 201 (M⁺,2), 147(32), 145 (100), 109 (17), 78 (8), 57,1 (17).

### Darstellung von Ligand 1 (α,α'-Bis(2-pyridyl(t-butyl)phosphino)o-xylen)

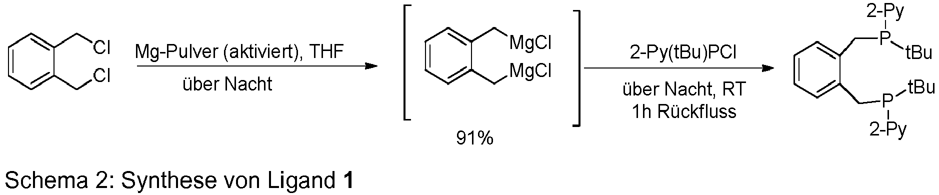

(Lit: Graham Eastham et al., Patent US 6335471)

675 mg (27,8 mmol, 4 eq) Mg-Pulver werden in der Glovebox in einem 250 ml-Rundkolben mit Stickstoffhahn und Magnetrührkern eingewogen und mit einem Septum verschlossen. Man legt an den Rundkolben Hochvakuum (ca. 5x10⁻² mbar) an und erwärmt für 45 Minuten auf 90 °C. Nach Abkühlen auf Raumtemperatur werden 2 Körnchen Iod zugegeben und in 20 ml THF gelöst. Man rührt die Suspension ca. 10 Minuten bis die gelbe Farbe des Iods verschwunden ist. Nach Absetzen des Magnesiumpulvers wird die trübe THF-Lösung dekantiert und das aktivierte Magnesiumpulver 2 Mal mit 1-2 ml THF gewaschen. Dann werden erneut 20 mL frisches THF zugegeben. Bei Raumtemperatur wird eine Lösung von 1,21 g (6,9 mmol) α,α'-Dichloro-o-xylene in 70 ml THF mit der Spritzenpumpe langsam zugetropft. Die THF-Lösung verfärbt sich langsam dunkel. Am nächsten Tag wird die THF-Suspension von dem nicht umgesetzten Magnesiumpulver filtriert und der Gehalt an Grignardverbindung wird wie folgt bestimmt:
1 ml Grignardlösung wird in einer gesättigten wässrigen Lösung von NH₄Cl gequencht und mit Ether extrahiert. Nach Trocknen über Na₂SO₄ wird von der Etherlösung ein GC aufgenommen. Man sieht qualitativ, dass ausschließlich o-Xylen entstanden ist.

### Quantitative Bestimmung von dem Gehalt der Grignardlösung:

1 ml Grignardlösung wird mit 2 ml 0,1 M HCL gequencht und die überschüssige Säure mit 0,1 M NaOH titriert. Als Indikator ist eine wässrige 0,04%ige Bromkresollösung geeignet. Farbumschlag geht von gelb nach blau. Es sind 0,74 ml an 0.1 M NaOH verbraucht worden. 2mL-0,74 ml = 1,26 ml, das entspricht 0,126 mmol Grignardverbindung. Da ein Digrignard vorliegt, ist die Grignardlösung 0,063 M. Das sind über 90% Ausbeute.

In einem 250mL Dreihalskolben mit Rückflusskühler und Magnetrührer werden unter Argon 1,8 g (8,66 mmol) Chlorphosphin (2-Py(tBu)PCI) in 10 ml THF gelöst und auf -60 °C gekühlt. Dann werden 55 ml der oben bestimmten Grignardlösung (0,063 M, 3,46 mmol) langsam bei dieser Temperatur mit der Spritzenpumpe zugetropft. Die Lösung bleibt zunächst klar und wird dann intensiv gelb. Nach 1,5 Stunden wird die Lösung trüb. Man lässt über Nacht auf Raumtemperatur aufwärmen und man erhält eine klare gelbe Lösung. Zur Vervollständigung der Reaktion erhitzt man 1 Stunde unter Rückfluss. Nach Abkühlen gibt man 1 ml H₂O dazu und die Lösung entfärbt sich und wird milchig trüb. Nach Entfernen von THF im Hochvakuum erhält man einen zähen, hellgelben Feststoff. Man gibt 10 ml Wasser und 10 ml Ether dazu und erhält zwei homogene klare Phasen, die sich gut trennen lassen. Die wäßrige Phase wird zweimal mit Ether extrahiert. Nach Trocknen der organischen Phase mit Na₂SO₄ wird der Ether im Hochvakuum entfernt und man erhält einen zähen fast farblosen Feststoff. Dieser wird in 5 mL MeOH unter Erwärmen auf dem Wasserbad gelöst und über Celite filtriert. Bei -28 °C erhält man über Nacht 772 mg Produkt in Form von weißen Kristallen. (51%). Aus der Mutterlösung konnten nach Einengen nochmals 100 mg isoliert werden. Die Gesamtausbeute beträgt 57,6 %.
¹H NMR (300 MHz, C₆D₆): δ 8,58 (m, 2H, Py), 7,31-7,30 (m, 2H, Benzol), 7,30-7,22 (m, 2H, Py), 6,85-6,77 (m, 2H, Py), 6,73 (m, 2H, Benzol), 6,57-6,50 (m, 2H, py), 4,33(dd, J = 13,3 und 4,3 Hz, 2H, CH₂), 3,72-3,62 (m, 2H, CH₂), 121(d, J = 11,8 Hz, 18H, tBu),
¹³C NMR (75 MHz, C₆D₆): δ 161,3, 161,1, 149,6, 137,8, 137,7, 134,5, 133,3, 132,7, 131,4, 131,3, 125,7, 122,9, 30,7, 30,5, 28,2, 28,0, 26,5, 26,4, 26,2, und 26,1.
³¹P NMR (121 MHz, C₆D₆) δ 8,8, EA berechnet für C₂₆H₃₄N₂P₂: C, 71,54; H, 7,85; N, 6,56; P,14,35, gefunden: C, 71,21; H, 7,55; N, 6,56; P, 14,35.

### Vergleichsligand

Als Vergleichsligand wird in den folgenden Beispielen 1,2-Bis(di-tert-butylphosphinomethyl)benzol (DTBMB, Ligand **3**) eingesetzt.

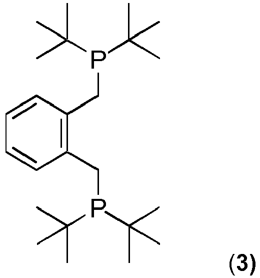

### Methoxycarbonylierung von tert-Butanol

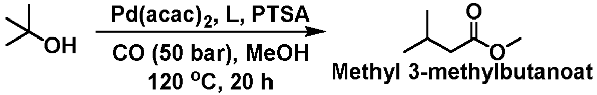

### 1) ohne Ligand (Vergleichsbeispiel)

Ein 4 ml-Reaktionsglasgefäß (Vial) wird unter Argon mit Pd(acac)₂ (3,1 mg, 0,5 mol%), PTSA (28,5 mg, 7,5 mol%) und einem Magnetrührer befüllt. Dann werden unter Argon MeOH (2 ml) und tert-Butanol (0,2 ml, 2 mmol) zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Die Ausbeute wurde mittels GC Analyse mit Isooctan (200 µl) als interner Standard durchgeführt (0% Ausbeute an Methyl 3-methylbutanoat).

### 2) Ligand 3 (Vergleichsbeispiel)

Ein 4 ml-Reaktionsglasgefäß (Vial) wird unter Argon mit Pd(acac)₂ (3,1 mg, 0,5 mol%), **3** (15,8, 2 mol%), PTSA (28,5 mg, 7,5 mol%, und einem Magnetrührer befüllt. Dann werden unter Argon MeOH (2 ml) und tert-Butanol (0,2 ml, 2 mmol) zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Die Ausbeute wurde mittels GC Analyse mit Isooctan (200 µl) als interner Standard durchgeführt (0% Ausbeute an Methyl 3-methylbutanoat).

### 3) Ligand 1

Ein 4 ml-Reaktionsglasgefäß (Vial) wird unter Argon mit Pd(acac)₂ (3,1 mg, 0,5 mol%), **1** (17,5 mg, 2 mol%), PTSA (28,5 mg, 7,5 mol%, und einem Magnetrührer befüllt. Dann werden unter Argon MeOH (2 ml) und tert-Butanol (0,2 mL, 2 mmol) zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Die Ausbeute wurde mittels GC Analyse mit Isooctan (200 µl) als interner Standard durchgeführt (99% Ausbeute an Methyl 3-methylbutanoat).

Die Ergebnisse sind in folgender Tabelle zusammengefasst:

| Beispiel | Ligand | Ausbeute an Methyl 3-methylbutanoat (%) |
|---|---|---|
| 1 (VB) | ohne | 0 |
| 2 (VB) | 3 | 0 |
| 3* | 1 | 99 |

| | | |
|---|---|---|
| VB: Vergleichsbeispiel *: erfindungsgemäßes Beispiel | | |

### Methoxycarbonylierung von 1-Phenyl-ethanol

Ein 4 ml-Reaktionsglasgefäß (Vial) wird unter Argon mit Pd(acac)₂ (3,1 mg, 0,5 mol%), PTSA (28,5 mg, 7 mol%), Ligand **1** (8,7 mg, 1 mol%) und einem Magnetrührer befüllt. Dann werden unter Argon MeOH (2 ml) und 2 mmol 1-Phenyl-ethanol zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Als Produkt wurde ein Gemisch aus Methyl-3-Phenylpropanoat (n-Produkt) und Methyl-2-Phenylpropanoat (iso-Produkt) erhalten. Die Ausbeute wurde mittels GC Analyse mit Isooctan (200 µl) als interner Standard durchgeführt (40% Gesamtausbeute, n/iso-Verhältnis 70/30).

### Methoxycarbonylierung von 3,3-Dimethylbutan-2-ol

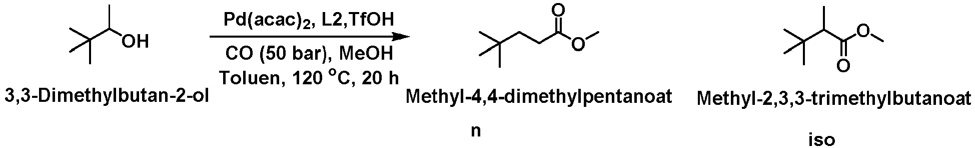

### Beispiel 1: Ligand 3

Ein 4 ml-Reaktionsglasgefäß (Vial) wird unter Argon mit Pd(acac)₂ (3,1 mg, 0,5 mol%), **3** (15,8, 2 mol%), CF₃SO₂OH (48,0 mg, 16 mol%) und einem Magnetrührer befüllt. Dann werden unter Argon Toluol (2 ml) und 3,3-Dimethylbutan-2-ol (0,25 ml, 2 mmol) zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Die Ausbeute wurde mittels GC Analyse mit Isooctan (200 µl) als interner Standard durchgeführt (0% Ausbeute).

### Beispiel 2: Ligand 3 mit 4 Å Molekularsieb

Ein 4 ml Reaktionsglasgefäß (Vial) wird unter Argon mit Pd(acac)₂ (3.1 mg, 0.5 mol%), **3** (15.8, 2 mol%), CF₃SO₂OH (48.0 mg, 16 mol%), 4Å Zeolith-Molekularsieb (4Å MS, 20 mg), und einem Magnetrührer befüllt. Dann werden unter Argon Toluol (2 ml) und 3,3-Dimethylbutan-2-ol (0,25 mL, 2 mmol) zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Die Ausbeute wurde mittels GC Analyse mit Isooctan (200 µl) als interner Standard durchgeführt (0% Ausbeute).

### Beispiel 3: Ligand 1

Ein 4 ml-Reaktionsglasgefäß (Vial) wird unter Argon mit Pd(acac)₂ (3,1 mg, 0,5 mol%), **1** (17,5mg, 2 mol%), CF₃SO₂OH (48,0 mg, 16 mol%) und einem Magnetrührer befüllt. Dann werden unter Argon Toluol (2 ml) und 3,3-Dimethylbutan-2-ol (0,25 ml, 2 mmol) zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Die Ausbeute wurde mittels GC Analyse mit Isooctan (200 µl) als interner Standard durchgeführt (10% Ausbeute).

### Beispiel 4: Ligand 1 mit 4 Å Molekularsieb

Ein 4 ml-Reaktionsglasgefäß (Vial) wird unter Argon mit Pd(acac)₂ (3,1 mg, 0,5 mol%), **1** (17,5mg, 2 mol%), CF₃SO₂OH (48,0 mg, 16 mol%) 4Å MS (20 mg), und einem Magnetrührer befüllt. Dann werden unter Argon Toluol (2 ml) und 3,3-Dimethylbutan-2-ol (0,25 ml, 2 mmol) zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Die Ausbeute wurde mittels GC Analyse mit Isooctan (200 µl) als interner Standard durchgeführt (20% Ausbeute).

Die Ergebnisse sind in folgender Tabelle zusammengefasst:

| Beispiel | Ligand | Additiv | Ausbeute | n/iso Verhältnis |
|---|---|---|---|---|
| 1 (VB) | **3** | | 0% | - |
| 2 (VB) | **3** | 4 Å MS | 0% | - |
| 3* | **1** | | 10% | 75/25 |
| 4* | **1** | 4 Å MS | 20% | 74/26 |

| | | | | |
|---|---|---|---|---|
| VB: Vergleichsbeispiele *: erfindungsgemäße Beispiele | | | | |

Die obigen Versuche zeigen, dass mit Hilfe des erfindungsgemäßen Verfahrens unter Verwendung der erfindungsgemäßen Liganden eine Reihe von strukturell unterschiedlichen Alkoholen als Substrat in einer Alkoxycarbonylierungsreaktion mit CO und einem weiteren Alkohol zu Estern umgesetzt werden können. Demgegenüber eignet sich der aus dem Stand der Technik bekannte Ligand nicht zur Umsetzung von Alkoholen in einer Alkoxycarbonylierungsreaktion.

## Patentansprüche

1. Verfahren umfassend die Verfahrensschritte:
a) Vorlegen eines ersten Alkohols, wobei der erste Alkohol 2 bis 30 Kohlenstoffatome aufweist;
b) Zugabe eines Phosphinliganden und einer Verbindung, welche Pd umfasst, oder Zugabe eines Komplexes umfassend Pd und einen Phosphinliganden;
c) Zugabe eines zweiten Alkohols;
d) Zuführen von CO;
e) Erwärmen des Reaktionsgemisches, wobei der erste Alkohol mit CO und dem zweiten Alkohol zu einem Ester umgesetzt wird;
wobei der Phosphinligand eine Verbindung gemäß Formel (**I**) ist 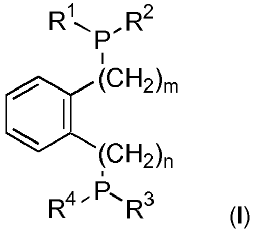 wobei
m und n unabhängig voneinander jeweils 0 oder 1 sind;
R¹, R², R³, R⁴ jeweils unabhängig voneinander ausgewählt sind aus -(C₁-C₁₂)-Alkyl,-(C₃-C₁₂)-Cycloalkyl, -(C₃-C₁₂)-Heterocycloalkyl, -(C₆-C₂₀)-Aryl, -(C₃-C₂₀)-Heteroaryl; mindestens einer der Reste R¹, R², R³, R⁴ für einen -(C₃-C₂₀)-Heteroarylrest steht; und
R¹, R², R³, R⁴, falls diese für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen,
jeweils unabhängig voneinander mit einem oder mehreren Substituenten ausgewählt aus
-(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können, und
wobei der erste Alkohol in Verfahrensschritt a) ein sekundärer oder tertiärer Alkohol ist und der zweite Alkohol in Verfahrensschritt c) ein primärer Alkohol ist,
hierbei umfasst der Begriff (C₃-C₁₂)-Heterocycloalkyl nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind.

2. Verfahren nach Anspruch 1,
wobei der Phosphinligand eine Verbindung gemäß einer der Formeln (**II**) und (**III**) ist

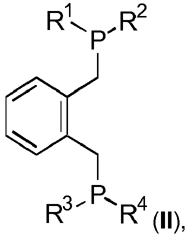

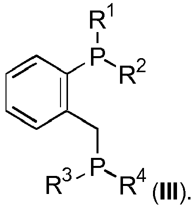

3. Verfahren nach einem der Ansprüche 1 bis 2,
wobei mindestens zwei der Reste R¹, R², R³, R⁴ für einen -(C₃-C₂₀)-Heteroarylrest stehen.

4. Verfahren nach einem der Ansprüche 1 bis 3,
wobei die Reste R¹ und R³ jeweils für einen -(C₃-C₂₀)-Heteroarylrest stehen.

5. Verfahren nach einem der Ansprüche 1 bis 4,
wobei die Reste R¹ und R³ jeweils für einen -(C₃-C₂₀)-Heteroarylrest stehen; und R² und R⁴ jeweils unabhängig voneinander ausgewählt sind aus -(C₁-C₁₂)-Alkyl, - (C₃-C₁₂)-Cycloalkyl, -(C₃-C₁₂)-Heterocycloalkyl, -(C₆-C₂₀)-Aryl.

6. Verfahren nach einem der Ansprüche 1 bis 5,
wobei die Reste R¹ und R³ jeweils für einen -(C₃-C₂₀)-Heteroarylrest stehen; und R² und R⁴ jeweils unabhängig voneinander ausgewählt sind aus -(C₁-C₁₂)-Alkyl.

7. Verfahren nach einem der Ansprüche 1 bis 6,
wobei R¹, R², R³, R⁴, falls diese für einen Heteroarylrest stehen, jeweils unabhängig voneinander ausgewählt sind aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

8. Verfahren nach einem der Ansprüche 1 bis 7,
wobei der Phosphinligand eine Verbindung gemäß Formel (**1**) ist

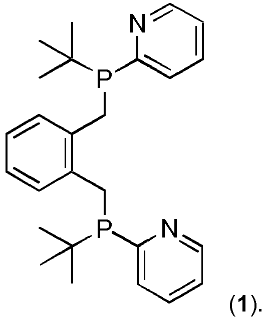

9. Verfahren nach einem der Ansprüche 1 bis 8,
wobei der erste Alkohol in Verfahrensschritt a) eine Verbindung gemäß Formel (**IV**) ist 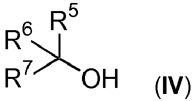
wobei R⁵ ausgewählt ist aus -(C₁-C₁₂)-Alkyl, -(C₃-C₁₂)-Cycloalkyl, -(C₆-C₂₀)-Aryl; und R⁶ und R⁷ jeweils unabhängig voneinander ausgewählt sind aus -H, -(C₁-C₁₂)-Alkyl, -(C₃-C₁₂)-Cycloalkyl, -(C₆-C₂₀)-Aryl,
wobei höchstens einer der Reste R⁶ und R⁷ -H ist.

10. Verfahren nach Anspruch 9,
wobei R⁵ -(C₁-C₁₂)-Alkyl ist.

11. Verfahren nach einem der Ansprüche 9 bis 10,
wobei R⁶ und R⁷ jeweils unabhängig voneinander ausgewählt sind aus -H, -(C₁-C₁₂)-Alkyl, -(C₆-C₂₀)-Aryl,
wobei höchstens einer der Reste R⁶ und R⁷ -H ist.

12. Verfahren nach einem der Ansprüche 1 bis 11,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

13. Verfahren nach einem der Ansprüche 1 bis 12,
wobei der zweite Alkohol in Verfahrensschritt c) ausgewählt ist aus Methanol, Ethanol, 1-Propanol, 1-Butanol, 1-Pentanol, 1-Hexanol, oder Mischungen davon.

## Claims

1. Process comprising the following process steps:
a) introducing a first alcohol, the first alcohol having 2 to 30 carbon atoms;
b) adding a phosphine ligand and a compound which comprises Pd,
or adding a complex comprising Pd and a phosphine ligand;
c) adding a second alcohol;
d) supplying CO;
e) heating the reaction mixture, the first alcohol reacting with CO and the second alcohol to form an ester;
where the phosphine ligand is a compound of formula (**I**) 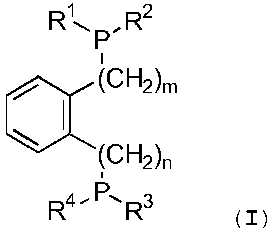 where
m and n are each independently 0 or 1;
R¹, R², R³, R⁴ are each independently selected from -(C₁₋C₁₂)-alkyl, -(C₃-C₁₂)-cycloalkyl, -(C₃-C₁₂)-heterocycloalkyl, -(C₆-C₂₀)-aryl, -(C₃-C₂₀)-heteroaryl;
at least one of the R¹, R², R³, R⁴ radicals is a-(C₃-C₂₀)-heteroaryl radical;
and
R¹, R², R³, R⁴, if they are -(C₁-C₁₂)-alkyl, -(C₃-C₁₂)-cycloalkyl, -(C₃-C₁₂)-heterocycloalkyl, -(C₆-C₂₀)-aryl or -(C₃-C₂₀)-heteroaryl,
may each independently be substituted by one or more substituents selected from
-(C₁-C₁₂)-alkyl, -(C₃-C₁₂)-cycloalkyl, -(C₃-C₁₂)-heterocycloalkyl, -O-(C₁-C₁₂)-alkyl, -O-(C₁-C₁₂)-alkyl-(C₆-C₂₀)-aryl, -O-(C₃-C₁₂)-cycloalkyl, -S-(C₁-C₁₂)-alkyl, -S-(C₃-C₁₂)-cycloalkyl, -COO-(C₁-C₁₂)-alkyl, -COO-(C₃-C₁₂)-cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -(C₆-C₂₀)-aryl, -(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen, and
where the first alcohol in process step a) is a secondary or tertiary alcohol and the second alcohol in process step c) is a primary alcohol,
where the expression (C₃-C₁₂)-heterocycloalkyl encompasses nonaromatic, saturated or partly unsaturated cycloaliphatic groups having 3 to 12 carbon atoms, where one or more of the ring carbon atoms are replaced by heteroatoms.

2. Process according to Claim 1,
where the phosphine ligand is a compound of one of the formulae (**II**) and (**III**)

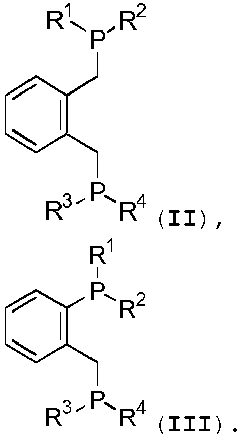

3. Process according to either of Claims 1 and 2,
where at least two of the R¹, R², R³, R⁴ radicals are a -(C₃-C₂₀)-heteroaryl radical.

4. Process according to any of Claims 1 to 3,
where the R¹ and R³ radicals are each a -(C₃-C₂₀)-heteroaryl radical.

5. Process according to any of Claims 1 to 4,
where the R¹ and R³ radicals are each a -(C₃-C₂₀)-heteroaryl radical;
and R² and R⁴ are each independently selected from-(C₁-C₁₂)-alkyl, -(C₃-C₁₂)-cycloalkyl, -(C₃-C₁₂)-heterocycloalkyl, -(C₆-C₂₀)-aryl.

6. Process according to any of Claims 1 to 5,
where the R¹ and R³ radicals are each a -(C₃-C₂₀)-heteroaryl radical;
and R² and R⁴ are each independently selected from-(C₁-C₁₂) -alkyl.

7. Process according to any of Claims 1 to 6,
where R¹, R², R³, R⁴, if they are a heteroaryl radical, are each independently selected from furyl, thienyl, pyrrolyl, oxazolyl, isoxazolyl, thiazolyl, isothiazolyl, imidazolyl, pyrazolyl, furazanyl, tetrazolyl, pyridyl, pyridazinyl, pyrimidyl, pyrazinyl, benzofuranyl, indolyl, isoindolyl, benzimidazolyl, quinolyl, isoquinolyl.

8. Process according to any of Claims 1 to 7,
where the phosphine ligand is a compound of formula (**1**)

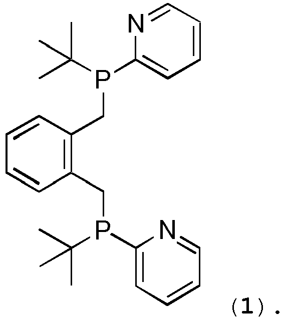

9. Process according to any of Claims 1 to 8,
wherein the first alcohol in process step a) is a compound of formula (**IV**) 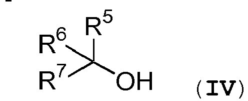
where R⁵ is selected from -(C₁-C₁₂) -alkyl, -(C₃-C₁₂)-cycloalkyl, -(C₆-C₂₀)-aryl;
and R⁶ and R⁷ are each independently selected from-H, -(C₁-C₁₂) -alkyl, -(C₃-C₁₂)-cycloalkyl, -(C₆-C₂₀)-aryl,
where not more than one of the radicals R⁶ and R⁷ is -H.

10. Process according to Claim 9,
wherein R⁵ is -(C₁-C₁₂) -alkyl.

11. Process according to any of Claims 9 to 10,
where R⁶ and R⁷ are each independently selected from -H, -(C₁-C₁₂) -alkyl, -(C₆-C₂₀)-aryl, where not more than one of the radicals R⁶ and R⁷ is -H.

12. Process according to any of Claims 1 to 11,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

13. Process according to any of Claims 1 to 12,
wherein the second alcohol in process step c) is selected from methanol, ethanol, 1-propanol, 1-butanol, 1-pentanol, 1-hexanol, and mixtures thereof.

## Revendications

1. Procédé comprenant les étapes de procédé suivantes :
a) le chargement d'un premier alcool, le premier alcool comprenant 2 à 30 atomes de carbone ;
b) l'ajout d'un ligand phosphine et d'un composé qui comprend du Pd,
ou l'ajout d'un complexe comprenant du Pd et un ligand phosphine ;
c) l'ajout d'un deuxième alcool ;
d) l'introduction de CO ;
e) le chauffage du mélange réactionnel, le premier alcool réagissant avec le CO et le deuxième alcool pour former un ester ;
le ligand phosphine étant un composé selon la formule (I) 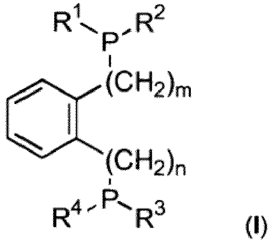 dans laquelle
m et n représentent chacun indépendamment l'un de l'autre 0 ou 1 ;
R¹, R², R³, R⁴ sont chacun choisis indépendamment les uns des autres parmi alkyle en (C₁-C₁₂), cycloalkyle en (C₃-C₁₂), hétérocycloalkyle en (C₃-C₁₂), aryle en (C₆-C₂₀), hétéroaryle en (C₃-C₂₀);
au moins un des radicaux R¹, R², R³ et R⁴ représentant un radical hétéroaryle en (C₃-C₂₀);
et
R¹, R², R³, R⁴, si ceux-ci représentent alkyle en (C₁-C₁₂), cycloalkyle en (C₃-C₁₂), hétérocycloalkyle en (C₃-C₁₂), aryle en (C₆-C₂₀) ou hétéroaryle en (C₃-C₂₀), pouvant chacun être substitués indépendamment les uns des autres avec un ou plusieurs substituants choisis parmi :
alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂), -CONH-alkyle en (C₁-C₁₂), -CONH-cycloalkyle en (C₃-C₁₂), -CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀)-O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀)-O-alkyle en (C₁-C₁₂), -COOH, -OH, -SO₃H, -NH₂, halogène, et
le premier alcool à l'étape de procédé a) étant un alcool secondaire ou tertiaire, et le deuxième alcool à l'étape de procédé c) étant un alcool primaire,
le terme hétérocycloalkyle en (C₃-C₁₂) comprenant ici les groupes cycloaliphatiques non aromatiques, saturés ou partiellement insaturés, de 3 à 12 atomes de carbone, un ou plusieurs des atomes de carbone de cycle étant remplacés par des hétéroatomes.

2. Procédé selon la revendication 1, dans lequel le ligand phosphine est un composé selon l'une quelconque des formules (II) et (III)

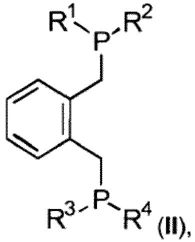

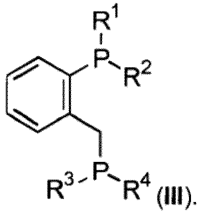

3. Procédé selon l'une quelconque des revendications 1 à 2, dans lequel au moins deux des radicaux R¹, R², R³, R⁴ représentent un radical hétéroaryle en (C₃-C₂₀).

4. Procédé selon l'une quelconque des revendications 1 à 3, dans lequel les radicaux R¹ et R³ représentent chacun un radical hétéroaryle en (C₃-C₂₀).

5. Procédé selon l'une quelconque des revendications 1 à 4, dans lequel les radicaux R¹ et R³ représentent chacun un radical hétéroaryle en (C₃-C₂₀) ; et R² et R⁴ sont chacun choisis indépendamment l'un de l'autre parmi alkyle en (C₁-C₁₂), cycloalkyle en (C₃-C₁₂), hétérocycloalkyle en (C₃-C₁₂), aryle en (C₆-C₂₀).

6. Procédé selon l'une quelconque des revendications 1 à 5, dans lequel les radicaux R¹ et R³ représentent chacun un radical hétéroaryle en (C₃-C₂₀) ; et R² et R⁴ sont chacun choisis indépendamment l'un de l'autre parmi alkyle en (C₁-C₁₂).

7. Procédé selon l'une quelconque des revendications 1 à 6, dans lequel R¹, R², R³, R⁴, si ceux-ci représentent un radical hétéroaryle, sont chacun choisis indépendamment les uns des autres parmi furyle, thiényle, pyrrolyle, oxazolyle, isoxazolyle, thiazolyle, isothiazolyle, imidazolyle, pyrazolyle, furazanyle, tétrazolyle, pyridyle, pyridazinyle, pyrimidyle, pyrazinyle, benzofuranyle, indolyle, isoindolyle, benzimidazolyle, quinolyle, isoquinolyle.

8. Procédé selon l'une quelconque des revendications 1 à 7, dans lequel le ligand phosphine est un composé selon la formule (1)

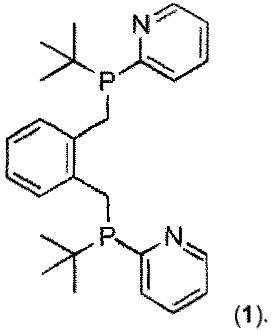

9. Procédé selon l'une quelconque des revendications 1 à 8, dans lequel le premier alcool à l'étape de procédé a) est un composé selon la formule (IV) 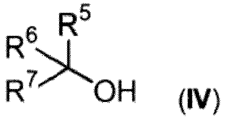
dans laquelle R⁵ est choisi parmi alkyle en (C₁-C₁₂), cycloalkyle en (C₃-C₁₂), aryle en (C₆-C₂₀) ;
et R⁶ et R⁷ sont chacun choisis indépendamment l'un de l'autre parmi -H, alkyle en (C₁-C₁₂), cycloalkyle en (C₃-C₁₂), aryle en (C₆-C₂₀),
au plus un des radicaux R⁶ et R⁷ représentant -H.

10. Procédé selon la revendication 9, dans lequel R⁵ représente alkyle en (C₁-C₁₂).

11. Procédé selon l'une quelconque des revendications 9 à 10, dans lequel R⁶ et R⁷ sont chacun choisis indépendamment l'un de l'autre parmi -H, alkyle en (C₁-C₁₂), aryle en (C₆-C₂₀),
au plus un des radicaux R⁶ et R⁷ représentant -H.

12. Procédé selon l'une quelconque des revendications 1 à 11, dans lequel le composé qui comprend du Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).

13. Procédé selon l'une quelconque des revendications 1 à 12, dans lequel le deuxième alcool à l'étape de procédé c) est choisi parmi le méthanol, l'éthanol, le 1-propanol, le 1-butanol, le 1-pentanol, le 1-hexanol ou leurs mélanges.
